# FASCICULE DE BREVET EUROPEEN

(11) **EP 1 628 683 B1**
(45) Date de publication et mention de la délivrance du brevet: **06.05.2009**
(21) Numéro de dépôt: 04742725.7
(22) Date de dépôt: 14.05.2004
(51) Int. Cl.: A61K 47/02, A61K 39/05, A61K 39/08, A61K 39/13

(54) **COMPOSITION VACCINALE COMPRENANT DU PHOSPHATE DE FER A TITRE D' ADJUVANT VACCINAL**
IMPFSTOFFZUSAMMENSETZUNG, EISENPHOSPHAT ALS ADJUVANS ENTHALTEND
VACCINE COMPOSITION COMPRISING IRON PHOSPHATE AS A PHARMACEUTICAL AID TO SAID VACCINE

(30) Priorité: 16.05.2003 FR 0305876
(43) Date de publication de la demande: 01.03.2006
(73) Titulaire: Sanofi Pasteur, 69367 Lyon Cedex 07 (FR)
(72) Inventeur: SAUZEAT, Elizabeth, F-69210 Lentilly (FR)
(74) Mandataire: Kerneis, Daniéle
(86) Numéro de dépôt international: PCT/FR2004/001175
(87) Numéro de publication internationale: WO 2004/103408

(56) Documents cités:
- GB-A- 876 027
- US-A- 5 252 327
- US-A- 5 895 653
- WARREN J ET AL: "The adjuvant effect of powdered ferric oxide: enhancement of response to Mycoplasma Pneumoniae and respiratory syncytial virus vaccines" JOURNAL OF IMMUNOLOGY, WILLIAMS & WILKINS CO, US, vol. 102, no. 5, 1969, pages 1300-1308, XP002111597 ISSN: 0022-1767
- GUPTA R K ET AL: "Adjuvants - a balance between toxicity and adjuvanticity" VACCINE, BUTTERWORTH SCIENTIFIC. GUILDFORD, GB, vol. 11, no. 3, février 1993 (1993-02), pages 293-306, XP002111596 ISSN: 0264-410X

## Description

La présente invention est relative au domaine des vaccins, et notamment au domaine des compositions vaccinales comprenant un adjuvant. Plus particulièrement, la présente invention concerne un vaccin dans lequel l'adjuvant comprend du phosphate de fer.

On connaît dans l'art antérieur des compositions vaccinales comprenant des adjuvants destinés à accroître ou à modifier qualitativement la réponse immunitaire induite lors de l'administration d'un antigène. En effet, les développements réalisés dans l'industrie du vaccin, conduisent à utiliser des antigènes obtenus à partir de virus ou de bactéries, mais avec des procédés de purification de plus en plus élaborés, ainsi que des antigènes issus de la biotechnologie. Ces antigènes présentent l'avantage d'être généralement plus purs que certains des antigènes utilisés dans le passé, mais en contrepartie, ils sont souvent moins immunogènes et nécessitent donc l'utilisation d'un adjuvant.
De nombreux adjuvants ont déjà été décrits: saponines, émulsions, lipides cationiques... etc.
Cependant, à ce jour, les seuls adjuvants couramment utilisés dans les produits commercialisés sont les adjuvants à base d'aluminium. Or, il serait souhaitable de pouvoir disposer d'autres adjuvants.
Parmi l'art antérieur relatif aux adjuvants, on peut citer notamment le brevet US5895653 qui décrit à titre d'adjuvant des composés d'hydroxyde de fer sous forme de solution colloïdale dont le pouvoir adsorbant serait amélioré par rapport aux hydroxydes de fer précédemment décrits sous forme de gel.
Cependant, de tels adjuvants, s'ils présentent une alternative aux adjuvants à base d'aluminium actuellement utilisés, ne peuvent pas toujours les remplacer efficacement, et il est donc souhaitable de pouvoir disposer d'autres composés, dont les caractéristiques sont telles qu'ils présentent à la fois une bonne tolérance vis-à-vis des organismes auxquels ils sont administrés, qu'ils peuvent être produits dans des conditions compatibles avec les contraintes de l'industrie pharmaceutique, et surtout qu'ils permettent d'accroître ou de modifier la réponse immunitaire aux antigènes vaccinaux de façon au moins aussi efficace que les composés à base d'aluminium actuellement utilisés.

Dans ce but, la présente invention a pour objet une composition vaccinale comprenant au moins un antigène vaccinal et au moins un adjuvant, caractérisée en ce que l'adjuvant comprend du phosphate de fer.
Selon une caractéristique particulière, le phosphate de fer est présent sous forme d'une suspension de particules dont la taille est comprise entre 0,01µm et 300µm, et plus particulièrement entre 1 et 40 µm, avec en particulier une grande proportion des particules dont la taille est d'environ 7 µm.
Selon un mode particulier de réalisation de la présente invention, le phosphate de fer est préparé à partir d'une solution de sel de fer et de sel de phosphate.
Une telle préparation permet d'obtenir un produit blanchâtre non cristallin assurant toutes les conditions de sécurité nécessaires pour une administration à des être humains, généralement en bonne santé.
De nombreux avantages de la présente invention apparaîtront au cours de la description qui va suivre en référence aux figures qui illustrent des résultats obtenus lors des tests décrits à l'exemple 3 ; la figure 1 représente les résultats en IgGl après une 1^{ère} immunisation alors que la figure 2 représente les résultats obtenus après la 2^{nde} immunisation; la figure 3 représente les résultats en IgG2a obtenus après la 1^{ère} immunisation alors que la figure 4 représente les résultats obtenus après la 2^{nde} immunisation.
Par composition vaccinale au sens de la présente invention, on entend une composition susceptible d'être administrée à l'homme ou à animal afin d'induire une réponse du système immunitaire; cette réponse du système immunitaire pouvant se traduire par une production d'anticorps ou simplement, par une activation de certaines cellules, notamment les cellules présentatrices d'antigènes, les lymphocytes T, les lymphocytes B. La composition vaccinale peut être une composition à visée prophylactique ou à visée thérapeutique, ou encore les deux.
Par antigène au sens de la présente invention, on entend tout antigène susceptible d'être utilisé dans un vaccin, qu'il s'agisse de germe entier ou de sous-unité et quelle que soit sa nature: peptide, protéine, glycoprotéine, polysaccharide, glycolipide, lipopeptide...etc. Il peut s'agir d'antigènes viraux, bactériens ou autres; le terme antigène comprend également les polynucléotides dont les séquences sont choisies pour coder les antigènes que l'on souhaite voir exprimés par les individus auxquels sont administrés les polynucléotides, dans le cas de la technique d'immunisation que l'on appelle immunisation ADN. Il peut également s'agir d'un ensemble d'antigènes, notamment dans le cas d'une composition vaccinale multivalente qui comprend des antigènes susceptibles de protéger contre plusieurs maladies et que l'on appelle alors généralement une combinaison vaccinale, ou dans le cas d'une composition qui comprend plusieurs antigènes différents pour protéger contre une seule maladie, ainsi que cela est le cas pour certains vaccins contre la coqueluche ou la grippe par exemple.
De particulièrement bons résultats ont notamment été obtenus avec une composition vaccinale comprenant des antigènes contre la diphtérie, le tétanos et la poliomyélite.

Aux fins de la présente invention, le phosphate de fer est un composé minéral inerte à base de phosphate de fer amorphe, cristallin ou pseudo-cristallin. Des essais très satisfaisants ont été obtenus avec un phosphate de fer amorphe.
Un tel composé est préparé à partir d'une solution de sel de fer et de sel de phosphate. Comme sel de fer, on peut notamment utiliser le citrate de fer ou de façon particulièrement avantageuse le chlorure de fer.
Comme sel de phosphate, on peut notamment utiliser le phosphate de sodium.
La préparation du phosphate de fer peut se faire par mélange simultané ou successif des deux composants de départ. L'ordre du mélange peut être soit ajout de chlorure de fer dans du phosphate de sodium ou à l'inverse, ajout de phosphate de sodium dans du chlorure de fer. La vitesse d'ajout peut être très rapide ou au contraire au goutte à goutte.
Pour l'obtention d'un phosphate de fer convenant aux besoins de l'invention, un rapport de 2 fois plus de sel de fer que de sel de phosphate a été noté comme satisfaisant.
De façon alternative, on peut préparer le phosphate de fer à l'aide d'un nébuliseur. Dans ce cas, on peut nébuliser le phosphate de sodium dans une solution de chlorure de fer ou inversement le chlorure de fer dans le phosphate de sodium.

Lors de la préparation des compositions vaccinales selon l'invention, le phosphate de fer peut être introduit dans la composition vaccinale au tout début de la formulation. Le phosphate de fer est alors dilué à une certaine concentration, puis les antigènes d'intérêt y sont ajoutés.
Alternativement, il est possible d'ajouter le phosphate de fer à un lyophilisat comprenant déjà la portion de la composition vaccinale qui contient les antigènes d'intérêt.
Selon l'invention, le phosphate de fer est présent dans la composition vaccinale, en quantité suffisante pour exercer une action adjuvante.
Dans le cas où il est le seul adjuvant utilisé, la dose de composition vaccinale administrée peut comprendre entre 0.2 et 1.4 mg de phosphate de fer.
Dans le cas où il est associé à un autre adjuvant, les quantités présentes peuvent être réduites, en fonction de l'effet adjuvant recherché et de la puissance des autres adjuvants présents.

Des essais de toxicité locale et systémique sur lapins ont montré que le phosphate de fer était aussi bien toléré que l'hydroxyde d'aluminium.

Les exemples qui suivent illustrent des modes de réalisation particuliers de la présente invention.

### Exemple 1 : Préparation de phosphate de fer et d'hydroxyde (oxyhydroxyde) de fer.

Le phosphate de fer est préparé par mélange de FeCl₃, 6H₂O à 0.5 M ( fourni par SIGMA sous la référence de lot 55H 1251) dans une solution de Na₂HPO₄, 2H₂O à 0.25 M (fourni par Merck sous la référence de lot FA004911). Les 2 produits sont mélangés dans les proportions suivantes: à une solution de 800ml d' H₂O ultrafiltrée comprenant 108,12g de FeCl₃, 6H₂O, on associe Il d'eau ultrafiltrée comprenant 44.5 g de Na₂HPO₄, 2H₂O. Le mélange est effectué à température ambiante.
On agite pendant 15 minutes au moyen d'un agitateur-tige puis on effectue des centrifugations successives avec lavage du précipité par de l'eau. Après chaque lavage, on détecte la présence d'ions chlorures grâce à un dosage des ions chlorure au nitrate d'argent.
Lorsqu'il n'y a plus d'ions chlorure , c'est -à-dire après 10 lavages, le précipité est repris en eau et autoclavé 1h à 118°C afin d'assurer sa stérilité.
La solution obtenue de cette façon est une solution de phosphate de fer contenant 16 g Fe/l.

On prépare de l'hydroxyde (oxyhydroxyde) de fer par mélange à température ambiante de citrate de fer 0.5 M et de NaOH 2M. A 100ml de citrate de fer on ajoute goutte à goutte de la NaOH jusqu'à obtention d'un pH de 8. De la même façon que pour la préparation du phosphate de fer, on agite pendant 15 minutes puis on effectue des centrifugations successives avec lavage à l'eau du précipité, et dosage des ions chlorures au nitrate d'argent. Lorsqu'on ne détecte plus d'ions chlorure, le précipité est repris en eau et autoclavé 1h à 118°C pour être stérile. La solution alors obtenue est une solution d'hydroxyde (oxyhydroxyde) de fer à 6,3 g Fe/l.

### Exemple 2: Préparation des compositions vaccinales

A partir des solutions préparées comme indiqué ci-dessus, d'une suspension aqueuse d'hydroxyde (oxyhydroxyde) d'aluminium ( fourni par Reheis ) comprenant 10,8 g d'Al/l, ainsi que d'une préparation de PTP( Protéine Tétanique Purifiée) à une concentration de 330 unités floculantes ou Lf/ml (soit une concentration en protéines de 0,9125g/l), on effectue les compositions suivantes :
- PTP seul : 840 µl H₂O + 120µl NaCl 9% + 240 PTP à 100 Lf/ml,
- PTP et AlOOH : 618 µl H₂O + 222 µl AlOOH à 10.8 g d'Al/l + 120µl NaCl à 9% + 240 PTP à 100 Lf/ml,
- PTP et FePO₄ : 690 µl H₂O + 150 µl FePO₄ à 16 g/l + 120µl d'une solution tampon PBS concentrée 10 fois ( soit 95 mM Na₂HPO₄.2H₂O)+ 240 PTP à 100 Lf/ml,
- PTP et FeOOH : 458 µl H₂O + 382 µl FeOOH à 6.3 g de Fe/l + 120µl d'une solution tampon PBS concentrée 10 fois + 240 PTP à 100 Lf/ml

### Exemple 3 : Dosage des anticorps antitétaniques.

On dispose de 4 groupes de 6 souris OF1 femelles âgées de 6 semaines.
On administre à chaque groupe de souris, l'une des formulations préparées à l'exemple 2.
L'administration est réalisée par voie intra-musculaire dans les quadriceps sous anesthésie, à raison de 2 injections de 50 µl aux jours J1 et J22. La quantité administrée est donc de 2 unités floculantes ou Lf de PTP et de 0,2 mg de minéral ( soit de l'aluminium, soit du fer) dans le cas des formulations comprenant un adjuvant.
A J15, on effectue sur chaque souris une prise de sang d'environ 300µl au sinus rétro-orbitaire.
A J36, les souris sont exsanguinées sous anesthésie, par section de la carotide.
Sur chacun des sérums correspondants aux sangs recueillis, on dose par la méthode ELISA les IgGI et les IgG2a spécifiques de la PTP.

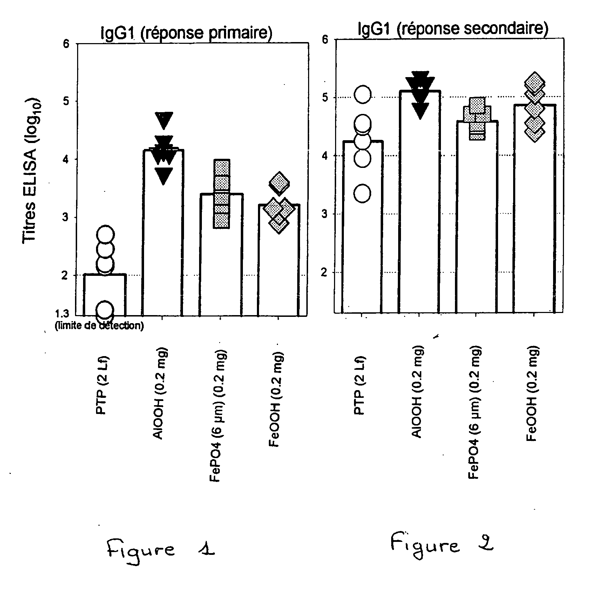

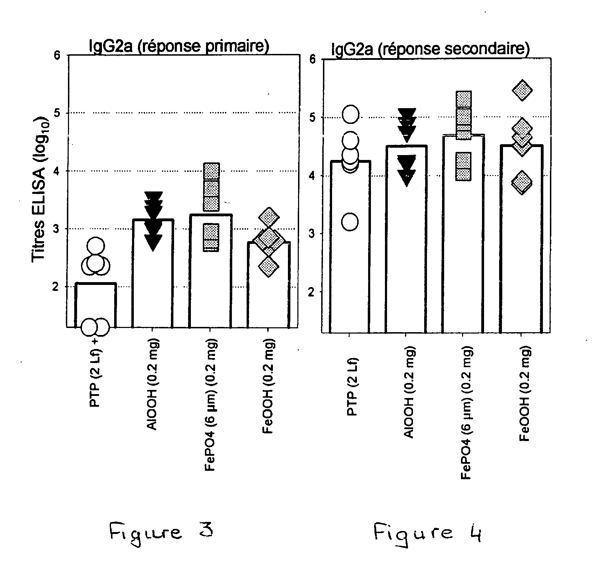

Les résultats obtenus sont indiqués aux figures 1 à 4 et montrent que le phosphate de fer est un bon adjuvant ; en ce qui concerne les IgGl, les résultats obtenus sont nettement supérieurs à ceux obtenus lorsque l'antigène est administré seul même s'ils sont légèrement inférieurs à ceux obtenus avec l'hydroxyde (oxyhydroxyde) d'aluminium; en ce qui concerne les IgG2a , les taux obtenus sont aussi élevés que ceux obtenus avec l'hydroxyde (oxyhydroxyde) d'aluminium.

### Exemple 4 : Vérification de l'activité antitétanique.

On vérifie l'activité antitétanique des compositions selon l'invention grâce à un test consistant à évaluer, chez la souris, l'activité de ces compositions vis à vis d'une dose létale de toxine administrée par voie sous-cutanée.

L'activité des compositions testées est déterminée en comparant la dose nécessaire pour protéger 50% des animaux (DE₅₀) des effets de la toxine à celle d'un vaccin de référence étalonné en UI/ml.

Les formulations testées sont obtenues par mélange de l'adjuvant (0.6 mg/dose) avec de l'eau, 30 Lf d'ADP (anatoxine diphtérique) et 10 Lf d'ATP (anatoxine tétanique), puis ajout de 20 µg/dose de merthiolate et une solution saline ( solution à 90 g/l NaCl et 5 mM Na₂HPO₄2H₂O).

Les compositions vaccinales testées comprennent soit de l'hydroxyde (oxyhydroxyde) d'aluminium, soit du phosphate de fer, soit de l'hydroxyde (oxyhydroxyde) de fer.
Les résultats obtenus avec chacun des adjuvants testés, sont repris dans le tableau ci-après, et montrent que le phosphate de fer est un bon adjuvant, bien veilleur que l'hydroxyde (oxyhydroxyde) de fer dans les mêmes conditions :

| adjuvants | Activité tétanique (UI/dose) |
|---|---|
| AlOOH | 103 (76-139) |
| FePO4 | 32(24-43) |
| FeOOH | 8.2 (5.4-12.5) |

## Revendications

1. Composition vaccinale comprenant au moins un antigène vaccinal et au moins un adjuvant, **caractérisée en ce que** l'adjuvant comprend du phosphate de fer.

2. Composition vaccinale selon la revendication précédente, **caractérisée en ce que** le phosphate de fer est présent sous forme d'une suspension de particules dont la taille est comprise entre 0,01 µm et 300 µm.

3. Composition vaccinale selon la revendication précédente, **caractérisée en ce que** la taille des particules est comprise entre 1 et 40 µm.

4. Composition vaccinale selon une des revendications précédentes, **caractérisée en ce que** la taille des particules est centrée autour de la valeur de 7µm.

5. Composition vaccinale selon une des revendications précédentes, **caractérisée en ce que** le phosphate de fer est préparé à partir d'une solution de sel de fer et de sel de phosphate.

6. Composition vaccinale selon une des revendications précédentes, **caractérisée en ce qu'**elle comprend au moins l'antigène du tétanos.

7. Composition vaccinale selon une des revendications précédentes, **caractérisée en ce qu'**elle comprend au moins l'antigène de la diphtérie.

8. Composition vaccinale selon une des revendications précédentes, **caractérisée en ce qu'**elle comprend au moins un antigène de la poliomyélite.

## Claims

1. Vaccine composition comprising at least one vaccine antigen and at least one adjuvant, **characterized in that** the adjuvant comprises iron phosphate.

2. Vaccine composition according to the preceding claim, **characterized in that** the iron phosphate is present in the form of a suspension of particles, the size of which is between 0.01 µm and 300 µm.

3. Vaccine composition according to the preceding claim, **characterized in that** the size of the particles is between 1 and 40 µm.

4. Vaccine composition according to one of the preceding claims, **characterized in that** the size of the particles is centered around a value of 7 µm.

5. Vaccine composition according to one of the preceding claims, **characterized in that** the iron phosphate is prepared from a solution of iron salt and of phosphate salt.

6. Vaccine composition according to one of the preceding claims, **characterized in that** it comprises at least the tetanus antigen.

7. Vaccine composition according to one of the preceding claims, **characterized in that** it comprises at least the diphtheria antigen.

8. Vaccine composition according to one of the preceding claims, **characterized in that** it comprises at least one poliomyelitis antigen.

## Patentansprüche

1. Impfstoffzusammensetzung, umfassend mindestens ein Impfantigen und mindestens ein Adjuvans, **dadurch gekennzeichnet, dass** das Adjuvans Eisenphosphat umfasst.

2. Impfstoffzusammensetzung nach dem vorstehenden Anspruch, **dadurch gekennzeichnet, dass** das Eisenphosphat in Form einer Suspension von Teilchen vorliegt, deren Größe zwischen einschließlich 0,01 µm und 300 µm beträgt.

3. Impfstoffzusammensetzung nach dem vorstehenden Anspruch, **dadurch gekennzeichnet, dass** die Größe der Teilchen zwischen einschließlich 1 µm und 40 µm beträgt.

4. Impfstoffzusammensetzung nach einem der vorstehenden Ansprüche, **dadurch gekennzeichnet, dass** die Größe der Teilchen um den Wert von 7 µm herum zentriert ist.

5. Impfstoffzusammensetzung nach einem der vorstehenden Ansprüche, **dadurch gekennzeichnet, dass** das Eisenphosphat ausgehend von einer Lösung von Eisensalz und Phosphatsalz hergestellt wird.

6. Impfstoffzusammensetzung nach einem der vorstehenden Ansprüche, **dadurch gekennzeichnet, dass** sie mindestens das Tetanus-Antigen umfasst.

7. Impfstoffzusammensetzung nach einem der vorstehenden Ansprüche, **dadurch gekennzeichnet, dass** sie mindestens das Diphtherie-Antigen umfasst.

8. Impfstoffzusammensetzung nach einem der vorstehenden Ansprüche, **dadurch gekennzeichnet, dass** sie mindestens ein Poliomyelitis-Antigen umfasst.
